# EUROPEAN PATENT SPECIFICATION

(11) **EP 1 617 915 B1**
(45) Date of publication and mention of the grant of the patent: **03.12.2008**
(21) Application number: 04727665.4
(22) Date of filing: 15.04.2004
(51) Int. Cl.: A61Q 19/00, A61K 8/49, A61K 36/00, A01N 65/00, C11D 3/382, A23L 3/3472

(54) **ACTIVATED CITRUS PEEL EXTRACT**
AKTIVIERTER CITRUSSCHALENEXTRAKT
EXTRAIT ACTIF D'ECORCE D'AGRUMES

(30) Priority: 15.04.2003 US 462740 P
(43) Date of publication of application: 25.01.2006
(73) Proprietor: Citramed Ltd., 78780 Ashkelon (IL)
(72) Inventor: MEDVEDEV, Anna, IL-84742 Beer Sheva (IL); KAT, Efrat, IL-69127 Tel Aviv (IL)
(74) Representative: Minoja, Fabrizio
(86) International application number: PCT/IL2004/000329
(87) International publication number: WO 2004/091569

(56) References cited:
- US-B1- 6 221 357
- DATABASE WPI Section Ch, Week 200142 Derwent Publications Ltd., London, GB; Class B04, AN 2001-392083 XP002297658 & IL 120 929 A (ISRAEL MIN AGRIC) 30 April 2001 (2001-04-30) cited in the application

## Description

### FIELD OF THE INVENTION

This invention relates to compositions containing activated citrus peel extract and uses thereof.

### BACKGROUND OF THE INVENTION

Since the beginning of mankind, harvesting of fruits and vegetables has played an important role in everyday life. Modem agriculture has allowed an increase in the amounts of harvested fruits and vegetables and due to lack of appropriate and efficient post-harvesting shipping and storage abilities for large quantities of such fruits and vegetables, has also caused increase in post-harvesting losses, which has become increasingly critical in countries where supply of fruits and vegetables is already low.

Citrus is one such crop. It is actually of the most common crops, and while so, the fruit peels are nearly completely unutilized and therefore discarded. The essential importance of citrus fruit peels has been realized in many fields of research relating to animal and human consumption. Furthermore, the utilization of the citrus fruit peels in the medicinal treatment of both animals and humans has also been realized.

The first thought that comes to mind when citrus fruit peels are mentioned is the oil which may be extracted therefrom by simply squeezing the peels or while the fruit is peeled. The production of this oil for medicinal or religious uses dates back to the eighteen century so that a considerable amount of data has been published about the various extraction methods and the various components identified therein.

Citrus peel extracts have been obtained and used for various applications. These uses are known to depend mostly on the natural activity of the peel extracts against various bacteria and fungi. The efficacy however of the extract on the various applications depends to a large extent on the methods of production. Many processes for the production of the oil have provided extracts which exhibited limited efficacy as the active component existed therein in small quantities.

One method of production of the citrus peel extract is described in Israel Patent No. 120929. This method comprises contacting citrus fruit peels with plant fungal and/or bacterial pathogens, incubating the citrus peels and aqueous extracting from them the active extract.

### SUMMARY OF THE INVENTION

The present invention is directed to an activated citrus peel extracts, herein designated ACPE, to the uses thereof and to compositions containing said extract.

The ACPE used in the compositions of the present invention is obtainable by a method which involves contacting of the citrus peels with at least one pathogen prior to extraction, said method hereon referred to as the "*activation method*". The extract obtained from such a method is hereon referred to as the "*activated extract*" or as ACPE.

The activation method may for example be the method disclosed in Israel Patent No. 120929 or the method of the present invention as disclosed hereinafter. The pathogens utilized for the activation of the citrus peels may be one or more plant or animal pathogens, preferably plant pathogens, selected from fungal or bacterial pathogens. A combination of pathogens may also be used in the activation process. Such combinations are mixtures of spores of at least one fungal or bacterial pathogen. Preferably the pathogens are selected from *Penicillium digitatum, Penicillium itallicum, Phytophtora citrophtora* and *Pseudomonas syringae.* Most preferably the pathogen is *Penicillium digitatum.*

The ACPE is further characterized as comprising one or more of the following: oligosaccharides, short peptides, flavonoid glycosides, fatty acids, and triglycerides. This ACPE has been shown to be effective as antimicrobial and antibacterial agent against a variety of plant or animal pathogens.

In one aspect of the present invention there is provided a composition comprising an activated citrus peel extract (ACPE) prepared by an activation method which includes exposure of citrus peels to at least one pathogen, said ACPE comprising one or more or a combination of all of the following: oligosaccharides, short peptides, flavonoid glycosides, fatty acids and triglycerides.

In one embodiment, the extract comprises at least 30-60% oligosaccharides, 1-10% short peptides, 10-30% flavonoid glycosides, 5-15% fatty acids, and 5-15% triglycerides. In a preferred embodiment, the extract comprises 50-60% oligosaccharides, 3-7% short peptides, 15-25% flavonoid glycosides, 8-12% fatty acids, and 8-12% triglycerides. In a most preferred embodiment, the extract comprises 55% oligosaccharides, 5% short peptides, 20% flavonoid glycosides, 10% fatty acids and 10% triglycerides.

The use of the symbol "%" or the term "***percent***" in the context of the ACPE extract will be understood to imply a weight proportion of each ingredient contained therein in relation to the weight of the whole extract (100%). For example, "10% fatty acids" refers to an extract containing 10g fatty acids in every 100g of extract (w/w proportion).

The composition of the present invention is a composition for preserving cosmetics, said composition comprises said activated citrus peel extract (ACPE).

In yet another aspect of the present invention there is provided a biocide composition which comprises ACPE for cleaning and disinfecting surfaces such as those found in households, hospitals, poultry and animal husbandry.

The process for the preparation of an activated citrus peel extract (ACPE) comprises:
(i) contacting citrus peels with spores of at least one fungal or bacterial pathogens, said pathogens being a 16 hour to 24 hour old bacteria or a 8 day to 14 old fungus,
(ii) incubating said citrus peels;
(iii) extracting the peels with water, and removing the peels from the aqueous liquid to obtain an aqueous extract.
(iv) filtering said aqueous extract through a membrane having a cutoff of between 800 and 2000 Da, and concentrating the extract to obtain said activated citrus peel extract.

In one embodiment, the process may include the adjustment of the pH of said un-concentrated aqueous extract obtained in step (iii) to a first pH of 8-10, filtrating it through a membrane having cutoff of between 800-2000 Da, readjusting its pH to second pH of 3-5, and concentrating the filtrate to obtain said activated citrus peel extract.

In one case, the pathogens are selected from *Penicillium digitatum, Penicillium itallicum, Phytophtora citrophtora* and *Pseudomonas syringae.* The pathogen is preferably *Penicillium digitatum*.

Also comprised within the scope of the present invention are the uses of the ACPE in the preparation of said compositions.

### DETAILED DESCRIPTION OF THE INVENTION

According to one aspect of the present invention, there is provided a composition comprising an ACPE produced by a method which involves activation of the citrus peels prior to the extraction process, and which results in an extract or active mixture having the following ingredients: oligosaccharides, short peptides, flavonoid glycosides, fatty acids and triglycerides.

Extract composition analysis utilizing separation methods such as various chromatographic separations and others known in the art, of various lots of extracts obtained under similar activation conditions showed variations in the relative quantity of each ingredient. All compositions showed identical or closely similar activity, both in terms of efficacy and selectivity towards certain microorganisms. Examples of such extract compositions are as follows:
**1.** 55% oligosaccharides, 5% short peptides, 20% flavonoid glycosides, 10% fatty acids and 10% triglycerides;
**2.** 46% oligosaccharides, 3% short peptides, 18% flavonoid glycosides, 16% fatty acids, and 17% triglycerides;
**3.** 35% oligosaccharides, 10% short peptides, 30% flavonoid glycosides, 10% fatty acids, and 15% triglycerides;
**4.** 60% oligosaccharides, 6% short peptides, 25% flavonoid glycosides, 5% fatty acids and 4% triglycerides;
**5.** 41% oligosaccharides, 10% short peptides, 19% flavonoid glycosides, 15% fatty acids, 12% triglycerides and 3% unidentified components;
**6.** 51% oligosaccharides, 9% short peptides, 25% flavonoid glycosides, 5% fatty acids and triglycerides (combined) and 10% unidentified components.

The term "*comprise*" or variations thereof will be understood to imply the inclusion of a stated integer or a group of integers but not the exclusion of any other integer or group of integers. The term "***composition***" as used within the scope of the present invention, refers to a composition which includes the ACPE and may also include other integers. The ACPE may include other integers which may originate from the type of citrus used, the age of the peels, freshness of the peels, possible prior exposure of the fruit and peels (before harvesting of the fruit) to natural pathogens, the type of pathogen used for the activation process, its age, period of exposure and other variables. Nevertheless, these other ingredients do not affect in any way the activity of the ACPE as herein described and exemplified. Specifically, an expression such as "*ACPE comprises one or more of the following: oligosaccharides, short peptides, flavonoid glycosides, fatty acids, and triglycerides*" will be understood to imply an extract that despite the exclusion of one or more of the listed integers or despite the inclusion of other integers, maintains its antibacterial or antimicrobial activity and exhibits no lowered or diminished such activity.

Notwithstanding the above, typically the composition of the present invention will comprise a combination of all of the hereinbefore listed integers.

The term "***Oligosaccharides***" refers to a sugar containing 8 to 15 monosaccharide units joint by glycosidic bonds.

The term "***peptide***" refers to compounds made up of two or more amino acids joint by covalent bonds. The term "***short peptides***" refers to peptides having at least 2 such amino acids and having molecular weights of less than 800 g/mole.

The term "***flavonoid***" refers generally to compounds having a C6-C3-C6 ring structure that is two aromatic rings linked together with a C3 segment which in most cases constructs a phenyl-benzpyran skeleton. Within the context of the present invention, the term includes also subclasses such as flavones, flavonols, flavanones, flavanols, anthocyanidines, isoflavonoids, and derivatives thereof. The flavonoids may or may not have hydroxyl groups and glycosidic linkages. Those having such linkages are referred to as "***flavonoid glycosides***"*.* Those lacking sugars are termed "flavonoid aglycones' and are too encompassed within the present definition. Also encompassed with in the term are open ring compounds that structurally maintain the benzpyran skeleton.

Non-limiting example of flavonoids are: apigenin, luteolin and luteolin-7-glycosides, artemetin, casticin, 5-hydroxy-3,6,7,4'-tetramethoxyflavone, rutin, tanetin, vitexin, hesperidin, naingin, hesperetin and naringin.

The term "***fatty acid***" refers to saturated or unsaturated organic acids having more than 4 carbon atoms and one carboxylic acid group. Non-limiting examples of fatty acids are: linoleic acid, myristic acid, oleic acid, palmitic acid, and stearic acid. The term "***triglyceride***" refers to fatty acid triesters of glycerol. The triglycerides may be of the same fatty acid or a mixture thereof and may or may not be fully substituted.

The expression "***at least one pathogen***" or "***at least one fungal or bacterial pathogen***" refers to pathogens that may be utilized for the activation of the citrus peels. These are plant pathogens, selected from fungal or bacterial pathogens. A combination of pathogens are used in the activation process. Preferably the pathogens are selected from *Penicillium digitatum, Penicillium itallicum, Phytophtora citrophtora* and *Pseudomonas syringae.* The pathogen is most preferably *Penicillium digitatum.*

According to one embodiment, the preservative composition is a preservative composition dermatological preparations for the treatment, alleviation and/or prevention of various skin conditions.

The terms "***prevention***", "***alleviation***" and "***treatment***" as used herein refer to the administering of an amount of the composition of the present invention which is effective to ameliorate undesired symptoms associated with a condition, to prevent the manifestation of such symptoms before they occur, to slow down the progression of the condition, slow down the deterioration of symptoms, to enhance the onset of remission period, slow down the irreversible damage caused by the condition, to delay the onset of said progressive stage, to lessen the severity or cure the condition, or to prevent the condition form occurring or a combination of two or more of the above.

In one embodiment, the skin condition may be associated with a bacterial or a fungal infection. In another embodiment, the skin condition may be a secondary condition, resulting from diseases and conditions *not* associated with a bacterial or a fungal infection of the skin. Secondary conditions may for example be wounds caused by such infections and other secondary conditions as mentioned hereinafter.

The term "***bacterial infection***" refers to a skin infection caused by one or more bacteria, such as: *Propionibacterium acnes*, *Entrococcus,* hemolytic *Streptococci, Staphylococci* and *M.R.S.A.* (Methicillin resistant Staphylococcus aureus), or a combination thereof. Examples of bacterial skin conditions in humans or animals are, without being limited to, acne, cellulites, folliculitis, boils (or carbuncles), Staphylococcal scalded skin syndrome, Erysipelas, Erythrasma, Impetigo and Paronychia.

The term "***fungal infection***" refers to a skin infection caused by one or more fungi such as *Canis, Trichophyton, Mentagraphtes, Rubrum, Violaceum, Epidermophyton, Icrosporum* and *Candida,* or a combination thereof. Examples of skin conditions associated with fungal infections are, without being limited to, ringworm, Candidiasis, Tinea Pedis and Tinea versicolor.

The compositions of the present invention are not harmful to the body of either humans or animals and thus varying concentrations of compositions may be used to achieve the desired effect.

According to an aspect of the invention, the ACPE composition is a composition for preserving cosmetics, or any other composition, substance or utility which may be susceptible to spoilage or decomposition as a result from exposure to various microorganisms, including bacteria, fungi, and the like. The composition comprises the ACPE of the present invention, having the constitution disclosed hereinbefore, and an acceptable carrier, excipient or diluent which is chosen in line with the expected utility of the composition.

The composition of the present invention may be incorporated into wrapping or containing materials such as those used to contain or hold fruits and vegetables. The ACPE composition contained within said wrapping or containing materials may be designed such as to allow slow or controlled release of the active extract from the wrapper or the container to the fruit or vegetable contained therein during storage or shipment The wrapping or containing materials may for example be pouches, plastic or paper bags, nylon sheets, polyester sheets, paper wrapping, plastic or other sealed containers, paper or plastic materials for hand or machine wrappings of fruits and vegetables, and the like.

In another embodiment, the preservative may be used to prolong the shelf-life of cosmetics, such as, but not limiting to, creams, ointments, gels, lotions, compositions for face masks and the like.

For prolonging produce shelf life, the ACPE preservative may be added during the preparation or treatment of the produce in sufficient amounts, thereby inhibiting or minimizing growth of microorganisms.

The preservative composition as with the other composition disclosed herein are able of being effective against a wide range of microorganisms such as *Salmonella sp., Staphylococci sp., Streptococci sp., Micrococci sp., E. Coli,* Coliform species, *Pseudomonas sp., Entrococci sp., Pasteurella sp., Alternaria spp., Fusarium spp., Penicillium spp., Cladosporium spp., Botrytis cinerea, Aspergillus niger,* and others hereinbefore and in the examples which follow.

In a further aspect of the present invention, the ACPE composition is a biocide composition for cleaning and disinfecting which comprises a surfactant and the ACPE of the present invention.

The term "***biocide composition***" refers to a composition containing ACPE which is capable of destroying a whole population of living microorganisms or any portion thereof. The term encompasses also disinfecting and sterilizing capabilities.

The surfactant is preferably selected from nonionic and cationic surfactants. The nonionic surfactant may, for example, be one or more selected from polyglycol ethers, polyalkylene glycol dialkyl ethers, and the addition products of alcohols with ethylene oxides and propylene oxides.

The cationic surfactant may be selected from various quaternary ammonium salts such as, but not limiting to octyl dimethyl benzyl ammonium chloride, octyl decyl dimethyl ammonium chloride, dioctyl dimethyl ammonium chloride, didecyl dimethyl ammonium chloride and dimethyl ethyl benzyl ammonium chloride, or mixtures thereof such as, but not limiting to, alkyl dimethyl benzyl ammonium chlorides and dialkyl dimethyl ammonium chlorides.

In one embodiment, the biocide composition may further comprise dyestuffs, perfumes, builder, chelating agents and corrosion inhibitors. The composition may be used to clean and disinfect surfaces such as ceramic tiles, PVC, porcelain, stainless steel, marble, silver and chrome to remove grease, wax, oil, dry paint and mildew and the like. As it has been demonstrated herein the ACPE containing compositions are non-toxic to the human skin or body. The detergent composition may therefore also be used as a laundry additive.

In another embodiment, the detergent is used in poultry and animal husbandry. As this detergent composition is able of being effective against a wide range of microorganisms such as *Salmonella sp., Staphylococci sp., Streptococci sp., Micrococci sp., E. Coli,* Coliform species, *Pseudomonas sp., Entrococci sp., Pasteurella sp., Alternaria spp., Fusarium spp., Penicillium spp., Cladosporium spp., Botrytis cinerea, Aspergillus niger,* it may be used as a one-detergent substitute for several conventional detergent compositions.

The biocide composition may be in a liquid or solid form depending on the specific utility. The detergent may also take the form of an aerosol spray, in which case, the composition is mixed with an appropriate propellant such as mist activators and sealed in an aerosol container under pressure.

In one specific embodiment, the biocide composition is absorbed in a towel or a cloth, thus providing a disinfectant towel that may be used as means of applying the composition to the various surfaces or may be used to disinfect the hands and skin of an individual.

The biocide composition may be further used for the treatment of water reservoirs such as, but not limiting to, water systems, cooling systems, swimming pool, natural and artificial water reservoirs, fisheries, water tanks, aquariums, and any other volume of water.

In one embodiment, the composition is added in a dry form to the water reservoir in an amount sufficient to control the growth of bacteria and fungi. In another embodiment, the dry composition is added to a water reservoir after being dissolved in an appropriate vehicle.

In addition to the antibacterial and antifungal uses described hereinbefore, the composition comprising ACPE, namely, the cosmetic composition and the compositions for prolonging shelf-life thereof may also be used as an antioxidant.

In accordance with yet another aspect of the present invention, and as already disclosed hereinabove, it has been found that a more efficient ACPE extract may be obtained when applying the following procedure. Whole fruit was washed with water and other suitable detergents and then dried. The dried fruits were next peeled, cut to desired size, placed in incubation containers and sprayed with a pathogen, preferably being a plant pathogen, most preferably being a 16-hour to 24-hour old bacteria or 8 to 14 day-old fungus suspension. In case of a fungus suspension, the spore concentration is preferably 4x10⁷ spores/ml. The containers were then sealed and left at room temperature for 4 days. The peels were then transferred into a heating vessel and heated in water at 70°C for 2 hours. Then, the aqueous solution was run through a colander and the peels were pressed to collect the extract.

At this stage in the process, excess water may be removed by evaporation, yielding an opaque extract having the desired activity as herein described. This extract may however be improved by treating the water solution, prior to evaporation of the water.

Thus, the pH of the aqueous solution was made basic and was next loaded on a filtration unit, preferably an ultra-filtration unit, fitted with a membrane having a cutoff of 800 to 2000 Da. The preferred membrane was one having a cutoff of 1000Da. A membrane having a cutoff of 1000 Da refers to a membrane that allows passage of molecules having molecular weight smaller or equal to 1000Da. Molecules with higher molecular weights would not pass the membrane and would therefore remain collected thereon.

It should be pointed out that such a filtration may be performed with any filtration unit known in the art and which a person skilled in the art would find suitable for the purpose of this procedure.

After 7 hours the filtration ended, the pH of the collected filtrate was reduced to the desired pH and the solution was concentrated to obtain the desired ACPE.

The high molecular weight fraction, which was left behind, showed no activity The ACPE, on the other hand, exhibited all the required characteristics.

The plant pathogens, which may be used in the process, may be anyone selected from a group of non-toxic pathogens. Preferably the pathogens are *Penicillium digitatum*, *Penicillium itallicum*, *Phytophtora citrophtora* and *Pseudomonus syringae*. Most preferably the pathogen is *Penicillium digitatum*.

The process may comprise a further step of sterilization and pasteurization of the extract obtained thereby. The sterilization or pasteurization may be on the aqueous solution containing the ACPE or on the ACPE itself and may follow procedures well known in the art.

### Examples

The following examples are intended to further illustrate the present invention without limiting the scope thereof as claimed.

### Example 1: A method for the Preparation of an activated citrus peel extract (ACPE)

The ACPE utilized in the compositions of the present invention may be prepared, for example by the method of Israel Patent No. 120929 or by the process of the present invention. The process of the present invention involves the following steps:
1. Whole fruit is washed with water, 70% sodium hypochlorite, and ethanol, rinsed again with water and then dried.
2. The dried fruits are next peeled, cut to desired size and placed in incubation containers.
3. Spore concentration of 4x10⁷ spores/ml (filtered spores) of 10 day-old *Penicillium digitatum* are sprayed onto the peels in a homogenous fashion and the containers are then sealed and left at room temperature (25°C) for 4 days.
4. Next, the peels were transferred into a heating vessel and were heated in water (5:1) at 70°C for 2 hours. Then, the aqueous solution was run through a colander and the peels were pressed to collect the extract.
5. The pH of the aqueous solution was now raised to 8 and was next loaded on an utrafiltartion unit fitted with a membrane having a cutoff of 1000Da. After the filtration ended, the pH of the collected filtrate was reduced to 3.5 and the solution was concentrated to obtain the desired ACPE.

### Example 2: Activity of the ACPE in comparison to other citrus peel extracts

The activity of the ACPE in inhibiting microbial and fungal growths was examined in comparison with the activity of other citrus peel extracts that were obtained by the following methods:

### Method 1: Extraction with ethanol:

Ethanol (95%, 2.5 L) was added to 500 grams of fresh grapefruit peels. The peels were extracted at 60°C for 5 hours. The extract thus obtained was filtered through a common colander and the filtrate was concentrated under vacuum to obtain 150 grams of a syrupy extract.

### Method 2: Extraction with propylene glycol:

The method described in Method 1 above utilizing a 10% solution of Propylene glycol in water (2.5 L).

### Method 3: Extraction with Glycerol:

The method described in Method 1 above utilizing 10% glycerol in water (2.5 L).

### Method 4: Extraction with water:

500 grams of fresh grapefruit peels were extracted in 2.5 L water at 70°C for 2 hours. The extract thus obtained was filtered through a common colander and the filtrate was concentrated under vacuum to obtain 150 grams of a syrupy extract.

### Method 5: Extraction using cold press:

The citrus extract was obtained by cold press according to known methods in the industry, producing an extract containing 37.4% TSS (Total soluble solids).

The activity of each of the citrus peel extracts obtained by any one of the above methods was compared with the ACPE extract according to the following "disc" method:

Disc Method: Differing quantities of the ACPE were dripped onto 13-mm paper discs and were then dried. The discs now absorbed with the ACPE, were placed at the center of a Petri dish in which a specific microorganism was grown. After 24 hours, the radius of the growth-inhibited area was measured (in mm) and recorded. After an additional 24 hours the measurement was repeated.

Distilled water was used as a negative control. As **Table 1** shows, the activity of the ACPE against bacterial and fungal growth was enhanced in comparison with the activity of other citrus extracts obtained with un-activated methods.

**Table 1: Activity of ACPE as compared to other citrus peel extracts.**

| **Method/Inhibition by ACPE, in mm** | ***E-Coli*** | ***Cladosporium*** |
|---|---|---|
| **1** | 4.8 | 1.5 |
| **2** | 4.5 | 1.7 |
| **3** | 2.5 | 1.8 |
| **4** | 3.8 | 2.2 |
| **5** | 2.5 | 2.3 |
| **ACPE** | 17.4 | 10.2 |

The activity of the ACPE obtained by the process of the present invention was also compared to the activity of the extract obtained by the process of Israel Patent No. 120929. A Serial dilution test showed that the ACPE prepared by the method of Example 1 was 4 times as active against *Cladosporium* as was the extract of Israel patent no. 120929 and twice as active against *E*. *coli* as compared to the extract of patent no. 120929.

### Example 3. ACPE activity against microorganisms in aqueous solution

In this experiment, 100g aqueous samples containing each 50% ACPE (w/v) were separately inoculated by one of the test organisms shown in Table 2. The inoculated containers were incubated at 25°C together with the un-inoculated samples that contained water only.

The number of surviving microorganisms was monitored periodically during a 4-week incubation period.

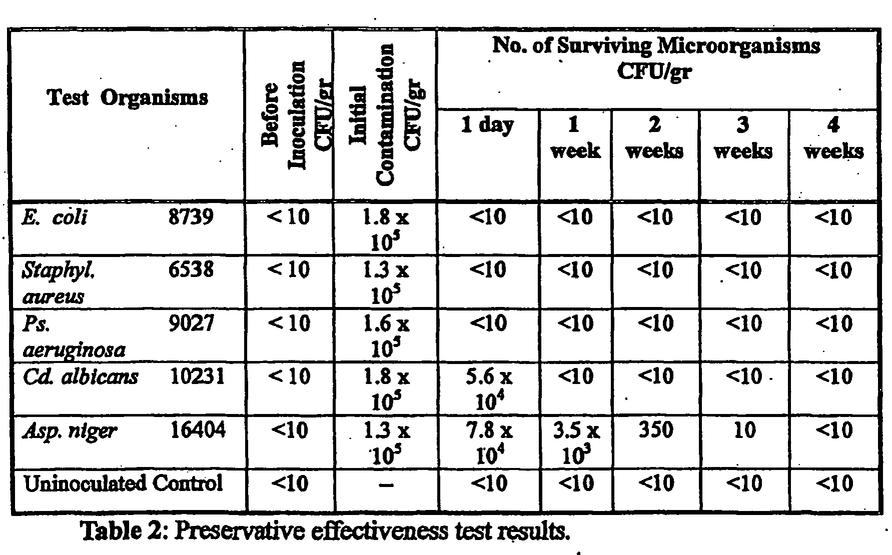

As **Table 2** shows, the efficiency of the ACPE in controlling the growth of the tested microorganisms was high. In the case of *E*. *Coli*, *Staphylococcus aureus* and *Pseudomonas aeruginosa* the ACPE completely inhibited growth within the first day of inoculation. With *Cd. Albicans* and *Asp. niger,* inhibition progressed over a period of 1-2 weeks.

### Example 4. ACPE as a preservative of dermatological formulations

Three different types of cosmetic cream having the hereinbefore exemplified ingredients (which previously did not contain any ACPE or other preservatives such as methyl paraben or propyl paraben) were mixed with 10-20% ACPE. Creams that did not contain any ACPE were used as controls. The controls were contaminated with fungus after several weeks. Creams which contained 10-20% ACPE were stable and showed no fungi related contamination for periods of several months (more than 3 months).

### Example 5. ACPE activity as an antimicrobial agent

The evaluation of the effectiveness of the ACPE against *Staphylococcus aureus, Streptococcus pyogenes* and *Trichophyton rubrum* (wild strain) was conducted at ACPE concentrations of 1%, 10%, 50% and 100% and at pH 3.5 and pH 5.

The sample dilutions were inoculated each one separately with a microbial load of 10⁵ cfu/ml of the challenge organisms, according to the guidelines set out in "Testing organisms and Preparation of Inoculum" USP 26.

The inoculated specimens were allowed to stand at room temperature for 6 and 24 hour periods. At the end of each period, 100 µl aliquots of each inoculated tube were transferred to an appropriate medium in separate Petri dishes. The media used were: Tryptic Soy Agar (TSA), Sabourand Dextrose Agar (SDA) plus Choramphenicol and Sterile Saline Solution. The TSA and SDA were also used for monitoring.

The number of cfu present in each test sample for the intervals was determined by the plate count procedure known to a person skilled in the art. By using the calculated concentrations of cfu per ml present at the start of the test, the change in log₁₀ values of the concentration of cfu per ml for each microorganism was calculated. This change is expressed below in terms of log reductions; for example, a -5log₁₀ reduction would mean a reduction of five orders of magnitude, for example from the 10⁵ microorganisms per ml starting load to less than 10 microorganism per ml. Such a log reduction is considered as a total eradication of the microorganisms' population and a total inhibition of any further growth.

For *Streptococcus pyogenes*, test solutions at pH 3.5 showed a total eradication of the microorganism's population. A -5log₁₀ reduction was exhibited for the initial calculated count at all concentrations at both 6 and 24 hours and was also achieved with the pH 5.0 up to the 10% solution concentration. The 1% solution showed a reduction of -1.64log₁₀ after 6 hours and of -5 log₁₀ after 24 hours.

For *Staphylococcus aureus,* test solutions containing 10%, 50% and 100% ACPE at pH 3.5 showed total eradication of the population up to the 50% concentration. The log₁₀ reductions for the 1% test solutions were -1.5log₁₀ and -5log₁₀ at 6 and 24 hours, respectively. The *Staphylococcus aureus* test solutions at pH 5.0 showed concordant results with a -5log₁₀ reduction at 100% and 50% concentrations and -3.6log₁₀ and -5log₁₀ at 10% concentration after 6 and 24 hours, respectively The 1% concentration test solution achieved a -0.6log₁₀ reduction.

Growth of *Trichophyton rubrum* was completely inhibited at the pH 3.5 test solutions at 100% and 50% concentrations. The 10% test solution showed -4log₁₀ and -5log₁₀ reductions after 6 and 24 hours, respectively . The 1% concentration solution showed -03log₁₀ and -0.6log₁₀ reductions after 6 and 24 hours; respectively. The inhibitory effect results of the pH 5.0 test solution correlated with the solution's concentration. The test solution at pH 5.0 showed a -5log₁₀ reduction after 24 hours at 100% and 50%. The 10% concentration test solution gave a reduction of -0.8log₁₀ after 24 hours and the 1% test solution achieved a -0.6log₁₀ reduction. Control testes in sterile water showed no log₁₀ reductions.

### Example 6: ACPE activity against food-associated microorganisms

In this experiment, 300 µL of ACPE solution containing 0.3 g ACPE per 1 ml of water was placed on 13-mm discs, which were positioned in the center of Petri dishes containing various food-associated microorganisms. As may be observed from **Table 4**, ACPE was shown to inhibit the growth of all 5 microorganisms.

**Table 4: Inhibition of food-associated bacteria**

| **Food Bacteria** | **ACPE Inhibition (diameter in mm)** |
|---|---|
| *E-Coli* 0157 | 16 |
| *Staphylococcus aureus* | 27 |
| *Streptococcus faecalis* | 17 |
| *Pseudomonas aeruginosa* | 17 |
| *Salmonella typhimurium* | 16 |
| *Listeria monocytogenes* | 23 |

The ACPE was active against all five strains of *Salmonella* tested: D-51234 pathogenic, C-51348, C-51119, C-51119 (Chicken run II) and C-51119 (Chicken Spleen). In addition, ACPE was active against the following strains of the *Pastorella* bacteria: 73204, 77745, 72262, 72784, and 78567.

The ACPE was also active in inhibiting the growth of the following strain of the *E-Coli* bacteria: 51460, 51461, and 51292.

## Claims

1. A process for the preparation of an activated citrus peel extract (ACPE) comprising:
**i)** contacting citrus peels with spores of at least one fungal or bacterial pathogens, said pathogens being a 16 hour to 24 hour old bacteria or a 8 day to 14 day old fungus;
**ii)** incubating said citrus peels;
**iii)** extracting the peels with water, and removing the peels from the aqueous liquid, **thereby obtaining an aqueous extract;**
**iv)** filtering said aqueous **extract through a membrane having a cutoff of between 800 and 2000 Da** and concentrating the **extract** to obtain said activated citrus peel extract.

2. The process according to claim **1**, wherein said pathogens are selected from *Penicillium digatatum, Penicillium itallicum, Phytophtora citrophtora* and *Pseudomonas syringae*.

3. The process according to claim **2**, wherein said pathogen is a 10-12 days old *Penicillium digatatum*.

4. An activated citrus peel extract (ACPE) obtainable by the process of any one of claims **1 to 3**.

5. The activated citrus peel extract according to claim **4** further comprising one or more oligosaccharides, short peptides, flavonoid glycosides, fatty acids, and triglycerides

6. The activated citrus peel extract according to claim **5** comprising at least 30-60% oligosaccharides, 1-10% short peptides, 10-30% flavonoid glycosides, 5-15% fatty acids, and 5-15% triglycerides.

7. Use of the activated citrus peel extract of any one of claims **4 to 6** for the preparation of a composition.

8. Use according to claim **7** wherein the composition is a preservative composition.

9. A composition comprising the activated citrus peel extract of any one of claims **4 to 6,** in admixture with an acceptable carrier, excipient or diluent.

10. A composition according to claim **8** which is a preservative composition.

11. Use of the preservative composition of claim **10** for preserving a composition, substance or utility which is susceptible to microbial spoilage or decomposition.

12. Use according to claim **9** wherein the composition susceptible to microbial spoilage is a cosmetic composition.

## Patentansprüche

1. Verfahren zur Herstellung eines aktivierten Zitrusschalenextrakts (ACPE), umfassend:
i) Inkontaktbringen von Zitrusschalen mit Sporen mindestens eines pilzlichen oder bakteriellen Krankheitserregers, wobei die Krankheitserreger ein 16 Stunden bis 24 Stunden altes Bakterium oder ein 8 Tage bis 14 Tage alter Pilz sind;
ii) Inkubieren der Zitrusschalen;
iii) Extrahieren der Schalen mit Wasser und Entfernen der Schalen aus der wässrigen Flüssigkeit, wodurch ein wässriger Extrakt erhalten wird;
iv) Filtern des wässrigen Extrakts durch eine Membran mit einen Ausschluss zwischen 800 und 2000 Da und Konzentrieren des Extrakts, um den aktivierten Zitrusschalenextrakt zu erhalten.

2. Verfahren nach Anspruch 1, wobei die Krankheitserreger aus *Penicillium digatatum, Penicillium itallicum, Phytophtora citrophtora* und *Pseudomonas syringae* ausgewählt sind.

3. Verfahren nach Anspruch 2, wobei der Krankheitserreger ein 10 bis 12 Tage alter *Penicillium digatatum* ist.

4. Aktivierter Zitrusschalenextrakt (ACPE), erhältlich nach dem Verfahren nach einem der Ansprüche 1 bis 3.

5. Aktivierter Zitrusschalenextrakt nach Anspruch 4, umfassend weiter ein oder mehrere Oligosaccharide, kurze Peptide, Flavonoid-Glycoside, Fettsäuren und Triglyceride.

6. Aktivierter Zitrusschalenextrakt nach Anspruch 5, umfassend mindestens 30-60% Oligosaccharide, 1-10% kurze Peptide, 10-30% Flavonoidglycoside, 5-15% Fettsäuren und 5-15% Triglyceride.

7. Verwendung des aktivierten Zitrusschalenextrakts nach einem der Ansprüche 4 bis 6 zur Herstellung einer Zusammensetzung.

8. Verwendung nach Anspruch 7, wobei die Zusammensetzung eine konservierende Zusammensetzung ist.

9. Zusammensetzung, umfassend den aktivierten Zitrusschalenextrakt nach einem der Ansprüche 4 bis 6 im Gemisch mit einem verträglichen Träger, Exzipienz oder Verdünnungsmittels.

10. Zusammensetzung nach Anspruch 8, die eine konservierende Zusammensetzung ist.

11. Verwendung der konservierenden Zusammensetzung nach Anspruch 10 zum Konservieren einer Zusammensetzung, einer Substanz oder eines Gebrauchsgegenstands, die bzw. der zum mikrobiellen Verderb oder Zersetzung neigt.

12. Verwendung nach Anspruch 9, wobei die Zusammensetzung, die zu mikrobiellem Verderb neigt, eine kosmetische Zusammensetzung ist.

## Revendications

1. Procédé pour la préparation d'un extrait d'écorce d'agrumes activé (ACPE), comprenant :
i) la mise en contact d'écorces d'agrumes avec des spores d'au moins un pathogène fongique ou bactérien, lesdits pathogènes étant une bactérie âgée de 16 heures à 24 heures ou un champignon âgé de 8 jours à 14 jours ;
ii) l'incubation desdites écorces d'agrumes ;
iii) l'extraction des écorces avec de l'eau, et l'enlèvement des écorces à partir du liquide aqueux, obtenant ainsi un extrait aqueux ;
iv) la filtration dudit extrait aqueux à travers une membrane laissant passer les molécules entre 800 et 2000 Da et la concentration de l'extrait pour obtenir ledit extrait d'écorce d'agrumes activé.

2. Procédé selon la revendication 1, dans lequel lesdits pathogènes sont choisis parmi *Penicillium digatatum*, *Penicillium itallicum, Phytophtora citrophtora* et *Pseudomonas syringae.*

3. Procédé selon la revendication 2, dans lequel ledit pathogène est un *Penicillium digatatum* âgé de 10 à 12 jours.

4. Extrait d'écorce d'agrumes activé (ACPE) susceptible d'être obtenu par le procédé selon l'une quelconque des revendications 1 à 3.

5. Extrait d'écorce d'agrumes activé selon la revendication 4, comprenant en outre un ou plusieurs oligosaccharides, peptides courts, glycosides de flavonoïdes, acides gras et triglycérides.

6. Extrait d'écorce d'agrumes activé selon la revendication 5, comprenant au moins 30 à 60 % d'oligosaccharides, 1 à 10 % de peptides courts, 10 à 30 % de glycosides de flavonoïdes, 5 à 15 % d'acides gras et 5 à 15 % de triglycérides.

7. Utilisation de l'extrait d'écorce d'agrumes activé selon l'une quelconque des revendications 4 à 6 pour la préparation d'une composition.

8. Utilisation selon la revendication 7, dans laquelle la composition est une composition de conservation.

9. Composition comprenant l'extrait d'écorce d'agrumes activé selon l'une quelconque des revendications 4 à 6, en mélange avec un véhicule, excipient ou diluant acceptable.

10. Composition selon la revendication 8, qui est une composition de conservation.

11. Utilisation de la composition de conservation selon la revendication 10 pour conserver une composition, une substance ou un matériel qui est susceptible d'une dégradation ou décomposition microbienne.

12. Utilisation selon la revendication 9, dans laquelle la composition susceptible à une dégradation microbienne est une composition cosmétique.
